# EUROPÄISCHE PATENTSCHRIFT

(11) **EP 1 371 331 B1**
(45) Veröffentlichungstag und Bekanntmachung des Hinweises auf die Patenterteilung: **10.08.2005**
(21) Anmeldenummer: 03011295.7
(22) Anmeldetag: 17.05.2003
(51) Int. Cl.: A61B 6/04

(54) **Röntgendiagnostikgerät für Mammographieuntersuchungen**
X-ray diagnosis apparatus for mammography
Appareil radiodiagnostique pour la mammographie

(30) Priorität: 13.06.2002 SE 0201806
(43) Veröffentlichungstag der Anmeldung: 17.12.2003
(73) Patentinhaber: Siemens Aktiengesellschaft, 80333 München (DE)
(72) Erfinder: Lindström, Krister, 125 34 Älvsjö (SE)

(56) Entgegenhaltungen:
- EP-A- 1 208 798
- EP-B- 0 370 089
- US-A- 5 335 257
- US-B1- 6 298 114

## Beschreibung

Die Erfindung betrifft ein Röntgendiagnostikgerät für Mammographieuntersuchungen mit einem Arm für eine Röntgenröhre und einem Objekttisch sowie einer zwischen der Röntgenröhre und dem Objekttisch angeordneten Kompressionsplatte, die mit dem Arm verbunden und entlang diesem verschiebbar ist, wobei der Arm über Verbindungsmittel mit einem Stativ drehbar verbunden ist.

Ein solches Röntgendiagnostikgerät ist durch die europäische Patentschrift 0 370 089 B1 bekannt. Bei einer Röntgenuntersuchung einer Brust eines Patienten ist das Ziel, eine so gute Bildqualität wie möglich bei einer niedrigst möglichen Röntgenstrahlendosis zu erreichen. Um dies zu erzielen, wird die zu untersuchende Brust komprimiert, indem sie zwischen dem Objekttisch und der Kompressionsplatte eingespannt wird. Dadurch, dass die Bruststärke geringer wird, kann auch die Röntgenstrahlendosis verringert werden. Die Kraft der Kompressionsplatte gegen die Brust ist in der Grössenordnung 10 bis 20 Kg, wodurch die Untersuchung mit Schmerz und Unannehmlichkeiten für den Patienten verbunden ist. Ein Teil des Schmerzes, der bei einer Kompression der Brust entsteht, kommt davon, dass die Brust häufig am Objekttisch und/oder an der Kompressionsplatte "festklebt", so dass leicht Falten auf der Brust entstehen. Ein Festkleben der Brust kann auch verhindern, dass sich die Brust gleichmäßig verteilt zwischen den Objekttisch und die Kompressionsplatte legt, was auch verhindert, dass eine optimale Verkleinerung der Bruststärke erreicht wird.

Der Erfindung liegt die Aufgabe zugrunde, ein Röntgendiagnostikgerät für Mammographieuntersuchungen der eingangs genannten Art zu schaffen, bei dem mit einfachen Mitteln eine optimale Kompression der Brust möglich ist, gleichzeitig damit, dass ein Reduzieren der Schmerzen bei der Kompression erreicht werden kann.

Diese Aufgabe wird erfindungsgemäß dadurch gelöst, dass das Röntgendiagnostikgerät mit Mitteln versehen ist, mit deren Hilfe ein Vibrieren der Kompressionsplatte erreicht werden kann.

In einer vorteilhaften Weiterbildung des Gerätes nach der Erfindung wird vorgeschlagen, dass das Mittel ausgebildet ist, sich in Verbindung mit einer Kompression so zu aktivieren, dass die Kompressionsplatte so lange vibriert, bis sie sich in ihrer Kompressionslage befindet. Mit Kompressionslage ist gemeint, dass die Kompressionsplatte eine Lage erreicht hat, bei der eine Aufnahme der Brust gemacht werden soll. Die Kompressionsplatte fängt vorzugsweise dann an zu vibrieren, wenn sie die Brust erreicht. Bei einer weiteren Kompression wird die Brust durch die Kompressionsplatte so beeinflusst, dass sie sich automatisch so gleich wie möglich verteilt an den Objekttisch legt, da die Brust durch die Vibration am Objekttisch und/oder an der Kompressionsplatte nicht festklebt. Dies verhindert auch, dass Hautfalten an der Brust entstehen. Die Vibration der Kompressionsplatte beeinflusst also die Stärke der Brust, so dass die Brust in einer Kompressionslage maximal dünn ist, was den Vorteil hat, dass die Röntgenstrahlendosis niedrig gehalten werden kann. Die Vibration hört auf, wenn sie die Kompressionslage erreicht hat.

Durch das Vibrieren der Platte bei einer Kompression der Brust ist ein weiterer Vorteil gegeben. Das Vibrieren kann nämlich ein Reizen der Brust hervorrufen, was mitsichbringt, dass das erwähnte üblicherweise vorkommende Gefühl von Unbehagen und vor allen Dingen der Schmerz bedeutend gelindert werden können.

Nach der Erfindung kann das Mittel so ausgebildet sein, dass die Kompressionsplatte seitlich oder in senkrechter Richtung vibriert.

In einer besonders vorteilhaften Weiterbildung des Gerätes nach der Erfindung wird vorgeschlagen, dass das Mittel derart ausgebildet ist, dass die Kompressionsplatte seitlich und in senkrechter Richtung vibriert.

Die erwähnten Mittel können motorgetriebene Vibratoren bekannter Art sein. Solche konventionellen Arten von Vibratoren werden u.a. in der US-Patentschrift 6 375 630 erwähnt.

Die Erfindung ist nachfolgend anhand eines in den Zeichnungen dargestellten Ausführungsbeispiels näher erläutert. Es zeigen:
- FIG 1: eine Seitenansicht eines Röntgendiagnostikgerätes nach der Erfindung und
- FIG 2: eine perspektivische Ansicht eines Teils eines Röntgendiagnostikgerätes nach FIG 1.

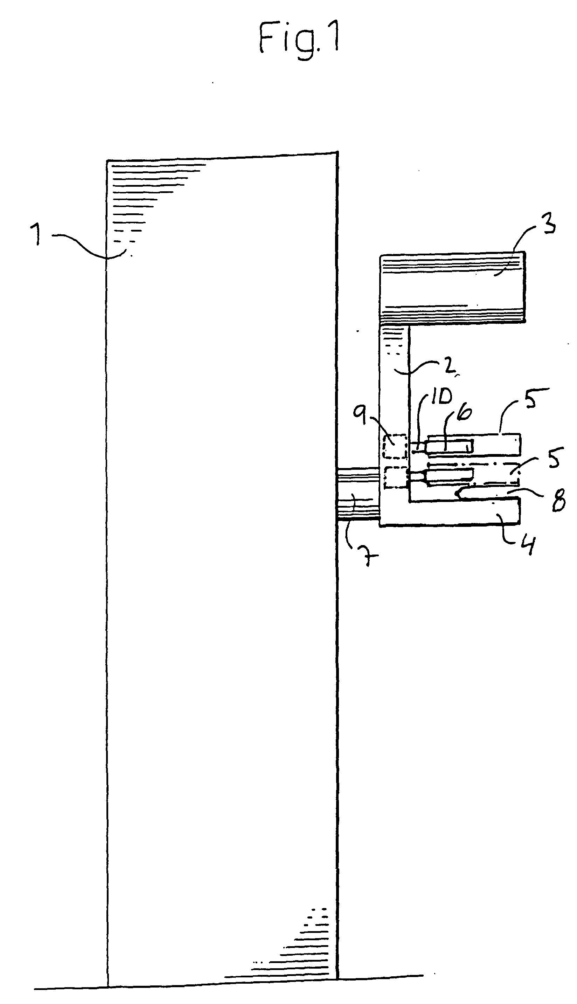

In der FIG 1 ist in schematischer Form ein Röntgendiagnostikgerät für Mammographieuntersuchungen gezeigt mit einem Stativ 1, das einen Arm 2 für eine Röntgenröhre 3 und einen Objekttisch 4 trägt. Zwischen der Röntgenröhre 3 und dem Objekttisch 4 ist eine Kompressionsplatte 5 angeordnet, die über eine Konsole 6 und über eine Welle 10 mit dem Arm 2 verbunden und entlang diesem verschiebbar ist. Der Arm 2 ist über eine horizontale Welle 7 mit dem Stativ 1 drehbar verbunden.

In der FIG 1 ist die Kompressionsplatte 5 teils in einer Parklage und teils in einer Kompressionslage gezeigt, bei der eine Brust 8 eines hier nicht gezeigten Patienten zwischen dem Objekttisch 4 und der Kompressionsplatte 5 komprimiert worden ist. Die zuletzt genannte Lage der Kompressionsplatte 5 ist angedeutet worden, in dem deren Konturen mit strichpunktierten Linien gezeichnet worden sind.

In Verbindung damit, dass die Kompressionsplatte 5 in ihre Kompressionslage verschoben wird, fängt die Platte 5 an mit Hilfe mindestens eines motorgetriebenen Vibrators, der z.B. im Arm 2 angebracht ist, zu vibrieren. Das Vibrieren erfolgt vorzugsweise dann, wenn die Platte 5 die Brust 8 erreicht bis die Platte 5 ihre optimale Kompressionslage erreicht hat. Somit wird vermieden, dass die Brust 8 am Objekttisch 4 oder an der Kompressionsplatte 5 festklebt, wobei die Brust 8 in vorteilhafter Weise am Objekttisch 4 verteilt wird, so dass eine optimale dünne Brust gegeben ist, wenn die Kompressionsplatte 5 ihre Kompressionslage erreicht hat. Eine optimal dünne Brust erlaubt, wie bereits erwähnt, eine optimal niedrige Röntgenstrahlendosis.

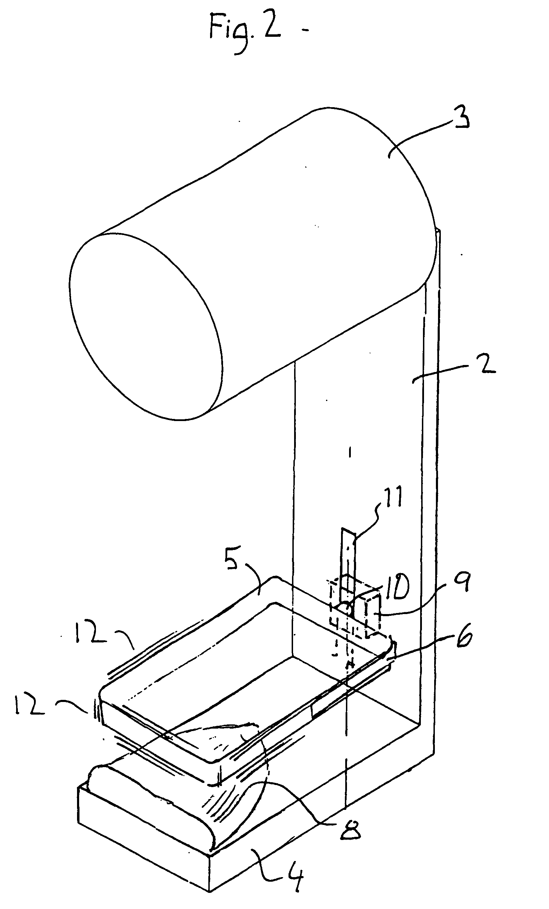

In der FIG 2 ist eine perspektivische Ansicht des Armes 2 mit der Röntgenröhre 3, dem Objekttisch 4 und der Kompressionsplatte 5 gezeigt. In der FIG ist dargestellt, dass die Welle 10 und damit die Konsole 6 der Kompressionsplatte 5 entlang einem am Arm 2 angebrachten Schlitz 11 verschiebbar ist.

Die Striche 12, die in dieser FIG um die Kompressionsplatte 5 gezogen sind, sollen ein Vibrieren der Platte 5 in Verbindung mit einem Komprimieren der Brust 8 darstellen.

Der motorgetriebene Vibrator 9 kann vorzugsweise so ausgebildet sein, dass die Kompressionsplatte 5 gleichzeitig seitlich und in senkrechter Richtung vibriert.

Der Vibrator 9 kann auch so ausgebildet sein, dass die Kompressionsplatte entweder seitlich oder in senkrechter Richtung vibriert.

Im Rahmen der Erfindung kann die Kompressionsplatte 5 bei einer Kompression mit Hilfe des Vibrators 9 anfangen zu vibrieren, wenn die Platte 5 ihre erwähnte Parklage verläßt, wobei ein Vibrieren kontinuierlich erfolgt, bis sie ihre optimale Kompressionslage erreicht hat.

## Patentansprüche

1. Röntgendiagnostikgerät für Mammographieuntersuchungen mit einem Arm (2) für eine Röntgenröhre (3) und einem Objekttisch (4) sowie einer zwischen der Röntgenröhre (3) und dem Objekttisch (4) angeordneten Kompressionsplatte (5), die mit dem Arm (2) verbunden und entlang diesem verschiebbar ist, wobei der Arm (2) über Verbindungsmittel (7) mit einem Stativ das Mittel (9) derart ausgebildet ist, dass drehbar verbunden ist, **dadurch gekennzeichnet, dass** das Gerät mit Mitteln (9) versehen ist, mit deren Hilfe ein Vibrieren der Kompressionsplatte (5) erreicht werden kann.

2. Röntgendiagnostikgerät nach Anspruch 1, **dadurch gekennzeichnet, dass** das Mittel (9) derart ausgebildet ist, dass die Kompressionsplatte (5) seitlich vibriert.

3. Röntgendiagnostikgerät nach Anspruch 1, **dadurch gekennzeichnet, dass** das Mittel (9) derart ausgebildet ist, dass die Kompressionsplatte (5) in senkrechter Richtung vibriert.

4. Röntgendiagnostikgerät nach Anspruch 1, **dadurch gekennzeichnet, dass** das Mittel (9) derart ausgebildet ist, dass die Kompressionsplatte (5) seitlich und in senkrechter Richtung vibriert.

5. Röntgendiagnostikgerät nach einem der Ansprüche 1 bis 4, **dadurch gekennzeichnet, dass** das Mittel (9) ausgebildet ist, sich in Verbindung mit einer Kompression so zu aktivieren, dass die Kompressionsplatte so lange vibriert, bis sie sich in ihrer Kompressionslage befindet.

## Claims

1. X-ray diagnostic device for mammogram examinations with an arm (2) for an x-ray tube (3) and a subject table (4) and a compression plate (5) arranged between the x-ray tube (3) and the subject table (4) which is connected to the arm (2) and which can be displaced along said arm (2), the arm (2) being rotatably connected to the stand (1) by a horizontal shaft (7), **characterised in that**
the device is provided with an apparatus (9) with the aid of which a vibration of the compression plate (5) can be achieved.

2. X-ray diagnostic device according to Claim 1, **characterised in that**
the apparatus (9) is configured such that the compression plate (5) vibrates laterally.

3. X-ray diagnostic device according to Claim 1, **characterised in that**
the apparatus (9) is configured such that the compression plate (5) vibrates vertically.

4. X-ray diagnostic device according to Claim 1, **characterised in that**
the apparatus (9) is configured such that the compression plate (5) vibrates laterally and vertically.

5. X-ray diagnostic device according to one of Claims 1 to 4, **characterised in that**
the apparatus (9) is configured to activate in conjunction with a compression such that the compression plate vibrates until it reaches its compression position.

## Revendications

1. Appareil de radiodiagnostic pour la mammographie comprenant un bras (2) pour un tube (3) radiogène et une tablette (4) pour un objet ainsi qu'une plaque (5) de compression interposée entre le tube (3) radiogène et la table (4) pour un objet, reliée au bras (2) et pouvant coulisser le long de celui-ci, le bras (2) étant relié de manière à pouvoir tourner à un statif (1) par des moyens (7) de liaison, **caractérisé en ce que** l'appareil est doté de moyens (9) à l'aide desquels on peut obtenir une vibration de la plaque (5) de compression.

2. Appareil de radiodiagnostic suivant la revendication 1, **caractérisé en ce que** les moyens (9) sont tels que la plaque (5) de compression vibre latéralement.

3. Appareil de radiodiagnostic suivant la revendication 1, **caractérisé en ce que** les moyens (9) sont tels que la plaque (5) de compression vibre en direction verticale.

4. Appareil de radiodiagnostic suivant la revendication 1, **caractérisé en ce que** les moyens (9) sont tels que la plaque (5) de compression vibre latéralement et en direction verticale.

5. Appareil de radiodiagnostic suivant l'une des revendications 1 à 4, **caractérisé en ce que** les moyens (9) sont tels qu'en liaison avec une compression ils peuvent être activés de façon à ce que la plaque de compression vibre jusqu'à ce qu'elle se trouve dans sa position de compression.
